Europäisches Patentamt

European Patent Office

Office européen des brevets

(11) Veröffentlichungsnummer: **0 450 490 A1**

## (12) EUROPÄISCHE PATENTANMELDUNG

(21) Anmeldenummer: 91104873.4

(22) Anmeldetag: 27.03.91

(51) Int. Cl.5: **A61F 13/20**

(30) Priorität: 03.04.90 DE 4010700

(43) Veröffentlichungstag der Anmeldung:
09.10.91 Patentblatt 91/41

(84) Benannte Vertragsstaaten:
AT BE CH DE DK ES FR GB GR IT LI LU NL SE

(71) Anmelder: HOECHST AKTIENGESELLSCHAFT
Postfach 80 03 20
W-6230 Frankfurt am Main 80(DE)

(72) Erfinder: Theis, Dirk, Dr.
Höchster Schlossplatz 4
W-6230 Frankfurt/a. Main(DE)
Erfinder: Fester, Walter, Dr.
Peter-Konrad-Strasse 24
W-8424 Saal(DE)

(54) Tampon und Verfahren zu dessen Herstellung.

(57) Beschrieben wird ein Tampon aus Endlosfilamenten auf Basis eines Chemiefaserkabels mit einem Wasserhaltevermögen von mindestens 1000 Gew.-%, der im wesentlichen aus senkrecht zur Tamponlängsachse angeordneten und durchgehenden Filamenten besteht und der im wesentlichen an der Oberfläche nur ein freies Filamentende pro Filament aufweist und das andere freie Filamentende in Innern enthält.

EP 0 450 490 A1

Die vorliegende Erfindung betrifft ein Tampon aus endlosen Filamenten und ein Verfahren zu dessen Herstellung. Tampons werden üblicherweise aus Vliesen, insbesondere aus Kardenvliesen hergestellt. Diese bestehen im allgemeinen aus Cellulosefasern.

Aus der US-A-4,627,849 sind Tampons bekannt, deren kennzeichnendes Merkmal im Aufbringen einer sogenannten "Mikrowelligkeit" besteht. Darunter ist eine Anordnung der gekräuselten Einzelfilamente zu verstehen, bei der die Knickpunkte mehrerer Filamente so zusammengefaßt sind, daß sich insgesamt eine Welligkeit der eingesetzten Fasermatten ergibt. Aus der Fig. 10a dieses Patents ist auch eine Ausführungsform bekannt, bei der ein Tampon erhalten werden kann, dessen Filamente vorzugsweise in Längsrichtung der Tamponachse verlaufen. Ein solcher Tampon ist allerdings aufwendig herzustellen. So ist beispielsweise eine kontinuierliche Herstellung nicht möglich. In diesem Patent wird auch die Verwendung endloser Faserkabel (in folgenden Kabel genannt) vorgeschlagen, doch wird diese Möglichkeit dort nicht näher ausgeführt.

Es wurde jetzt gefunden, daß man Tampons ausgehend von Kabeln in einfacher Weise herstellen kann. Es entfallen mit diesem Verfahren die Schritte der textilen Vorbehandlung, die durch die Verwendung von Kardenvliesen nötig waren und es ist eine kontinuierliche Herstellung möglich. Ferner erhält man ein Produkt, das praktisch keine freien Filamentenden entlang der Manteloberfläche des Tampons aufweist. Solche freien Filamentenden können zu Hautreizungen und allergischen Reaktionen führen und sind daher auf der Oberfläche des Tampons unerwünscht.

Die vorliegende Erfindung betrifft einen Tampon gemäß der in Anspruch 1 gegebenen Definition.

Das erfindungsgemäß eingesetzte Fasermaterial muß ein Wasserhaltevermögen (nach DIN 61640) von mindestens 1000 Gew.-% besitzen, d.h. es muß mindestens eine Wassermenge halten können, die mindestens dem Zehnfachen seines Eigengewichts entspricht. Üblicherweise beträgt das Wasserhaltevermögen 1000 bis 2000 Gew.-%, vorzugsweise jedoch mindestens 1500 Gew.-%.

Bei dem Fasermaterial kann es sich um Cellulose handeln, vorzugsweise um nicht modifizierte Cellulose. Beispiele dafür sind nach dem Direktlösungsverfahren ersponnene Cellulose, z.B. aus N-Methylmorpholin-N-oxid als Lösungsmittel ersponnene Cellulose, Regeneratcellulose, wie Kupferseide oder insbesondere Viskose. Das eingesetzte Kabel besitzt eine Stärke von etwa 5 bis 50 ktex, insbesondere von etwa 10 bis 40 ktex. Die Auswahl der Kabelstärke hängt im wesentlichen von den gewünschten Dimensionen des Endproduktes ab.

Der Einzelfadentiter des zu verwendenden Kabels liegt üblicherweise zwischen etwa 1 und 10 dtex, insbesondere zwischen etwa 2 bis 5 dtex. Die Auswahl dieser Größe wird nach oben durch die nötige mechanische Festigkeit des Tampons vorgegeben, während die untere Grenze hauptsächlich durch die Wirtschaftlichkeit des Faserherstellungsverfahrens bestimmt wird.

Das einzusetzende Kabel muß eine Kräuselung aufweisen, um dem Endprodukt eine ausreichende Festigkeit zu verleihen. Es können alle an sich üblichen Arten der Kräuselung angewendet werden, wie Zahnradkräuselung und insbesondere Stauchkammerkräuselung. Insbesondere bei Viskosefilamenten kann die durch den Viskoseprozeß bedingte Käuselung ohne weitere mechanische Behandlung ausreichen.

Wesentliche Merkmale des erfindungsgemäßen Tampons sind der Aufbau aus Endlosfilamenten der im wesentlichen senkrechte Verlauf dieser Filamente zur Längsaches des Tampons und der Einbau einer Hälfte der freien Filamentenden in das Innere des Tampons. Dadurch wird es möglich, daß nur die Hälfte der freien Filamentenden des Kabels sich auf der Mantelfläche des Zylinders befindet, so daß die später mit der Haut in Kontakt tretende Zylinderfläche nur einen geringen Gehalt an solch freien Filamentenden aufweist.

Der erfindungsgemäße Tampon kann direkt aus dem Kabelmaterial hergestellt werden oder er kann zusätzlich mit einem Hüllvlies versehen werden.

Solche Hüllvliese sind an sich bekannt. Dabei kann es sich beispielsweise um Ultraleichtvliese auf Basis von Polyester/Polyethylen Bikomponentenfasern handeln. Das Flächengewicht solcher Hüllvliese beträgt vorzugsweise weniger oder gleich 15 g/m$^2$, besonders bevorzugt etwa 10 g/m$^2$.

Der erfindungsgemäße Tampon kann mit einem Rückholfaden versehen werden.

Bei dem Rückholfaden kann es sich um Zwirne oder Garne aus natürlichen und/oder synthetischen Fasern handeln. Insbesondere verwendet man ein Garn oder einen Zwirn aus thermoplastischem Polymer, das nach dem Einbringen in den Tampon mit den es umgebenen Filamenten verklebt werden kann, beispielsweise durch Erhitzen über den Erweichungspunkt des thermoplastischen Polymeren.

Die Herstellung der erfindungsgemäßen Tampons erfolgt wie in Anspruch 8 definiert.

Das Kabel wird dazu in trapezförmige oder insbesondere in rechteckige Stücke geschnitten. Die Längsseiten dieser Stücke sind etwa 5 bis 30 cm lang, insbesondere etwa 10 bis 20 cm. Die Länge dieser Seiten bestimmt zusammen mit der Kabelstärke hauptsächlich den Durchmesser des erfindungsgemäßen Tampons. Die Breite des trapez- oder rechteckigen Kabelabschnitts bestimmt hauptsächlich die Länge des Tampons. Vorzugsweise wählt man Breiten zwischen 2 und 6 cm. Das Kabel kann vor dem Zurechtschneiden gegebenenfalls noch aufgefächert werden, um den Verlauf der Einzelfäden zu vergleichmässigen. Das Auffächern erfolgt in an sich bekannter Weise entweder mechanisch oder mittels Fluidstrahlen, wie z.B. in der US-A-3,156,016 oder der DE-A-1,435,417 beschrieben.

Nach dem Zurechtschneiden des Kabels kann der Tampon entweder sofort gerollt werden oder es wird zunächst ein Rückholfaden eingebracht. Dies erfolt durch Einlegen, Heften oder Befestigen eines Rückholfadens etwa in der Mitte der Längsseite des rechteckigen Kabelabschnittes, so daß der Faden im wesentlichen senkrecht zur Lage der Einzelfilamente verläuft.

In einer besonders bevorzugten Ausgestaltung des erfindungsgemäßen Verfahrens befestigt man den Rückholfaden an dieser Stelle durch Umschlingen des Kabelabschnitts mit dem Faden, wobei ein freies Ende des Fadens als Rückholfaden über eine Seite des Kabelabschnittes hinausragt.

Nach dem Schneiden und gegebenenfalls Einbringen eines Rückholfadens wird der Kabelabschnitt aufgerollt, wobei mit dem Rollen an einer Breitseite des Kabelabschnitts begonnen wird, so daß die freien Filamentenden dieser Seite sich im Innern des Tampons befinden. Nur die freien Filamentenden der anderen Breitseite des Kabelabschnitts befinden sich später auf der Oberfläche des Tampons. Diese Filamentenden verlaufen dabei, je nach Form des Kabelabschnitts, entweder linienförmig und schräg über die Tamponoberfläche oder bevorzugt linienförmig und in Richtung der Tamponlängachse über dessen Oberfläche.

Das aufgerollte Kabel wird anschließend verpresst, indem man senkrecht zur Zylinderoberfläche einen Druck aufbringt.

Eine geeignete Vorrichtung dafür ist z.B. in der Fig. 4, 4a und 4b der US-A-4,627,849 beschrieben. Vor dem Verpressen kann der Tampon noch mit einem Hüllvlies umwickelt werden. Verwendet man einen Rückholfaden aus thermoplastischen Material, so kann der Tampon während des Verpressens erhitzt werden, um den Rückholfaden mit den umgebenden Fasern zu verkleben. Dazu wird im allgemeinen eine Temperatur über dem Erweichungspunkt des thermoplastischen Materials benötigt.

Die erfindungsgemäßen Tampons sind vielseitig einsetzbar, beispielsweise für medizinische Zwecke oder insbesondere für die Frauenhygiene. Sie zeichnen sich durch eine hohe Saugkraft und durch eine besonders gute Verträglichkeit aus. Insbesondere sind sie auf einfache wirtschaftliche Weise herzustellen.

**Patentansprüche**

1.  Tampon aus Endlosfilamenten bestehend im wesentlichen aus einem Kabel aus gekräuselten Chemiefasern, welches ein Wasserhaltevermögen von mindestens 1000 Gew.-% besitzt, wobei das Kabel eine Stärke von etwa 5 bis 50 ktex und die Filamente in diesen Kabel einen Einzelfadentiter von etwa 1 bis 10 dtex aufweisen, und der Tampon im wesentlichen aus senkrecht zu dessen Längsachse angeordneten und durchgehenden Filamenten besteht, wobei pro Filament im wesentlichen nur ein freies Ende sich auf der Tamponoberfläche befindet und das andere freie Ende sich im Innern des Tampons befindet.

2.  Tampon gemäß Anspruch 1, dadurch gekennzeichnet, daß es sich bei den Chemiefasern um Cellulosefasern handelt.

3.  Tampon gemäß Anspruch 2, dadurch gekennzeichnet, daß es sich bei den Cellulosefasern um Viskosefasern handelt.

4.  Tampon gemäß einem der Ansprüche 1 bis 3, dadurch gekennzeichnet, daß der Einzelfadentiter etwa 2 bis 5 dtex beträgt.

5.  Tampon gemäß einem der Ansprüche 1 bis 4, dadurch gekennzeichnet, daß die sich auf der Oberfläche befindenen freien Enden der Filamente eine Linie bilden, die parallel zur Längsachse des Tampons verläuft.

6.  Tampon gemäß einem der Ansprüche 1 bis 5, dadurch gekennzeichnet, daß dieser mit einem Rückholfaden aus thermoplastischem Material versehen ist, der im Tampon mit den ihn umgebenden Filamenten verklebt ist.

7.  Tampon gemäß einem der Ansprüche 1 bis 6, dadurch gekennzeichnet, daß dieser zusätzlich mit einem Hüllvlies versehen ist.

8.  Verfahren zur Herstellung eines Tampons, gemäß Anspruch 1, ausgehend von einem Kabel aus gekräuselten Chemiefasern, welches ein Wasserhaltevermögen von mindestens 1000 Gew.-% besitzt, das eine Kabelstärke von etwa 5 bis 50 ktex besitzt und dessen Filamente einen Einzelfadentiter von etwa 1 bis 10 dtex aufweisen, umfassend die Schritte:

    i) Zurechtschneiden des Kabels in rechteckige oder trapezförmige Abschnitte der Länge von etwa 5 bis 30 cm und einer Breite, die etwa der gewünschten Tamponlänge entspricht, wobei die Einzelfilamente im wesentlichen in Längsrichtung verlaufen,

ii) Aufrollen des Kabelabschnittes beginnend an einer Breitseite dieses Abschnitts, an dem sich die freien Enden der Einzelfilamente befinden, und

iii) Verpressen des so erhaltenen Zylinders durch senkrechtes Aufbringen von Druck auf die Zylinderoberfläche, um dem Tampon die gewünschte mechanische Festigkeit und die gewünschte Stärke zu verleihen.

9. Verfahren zur Herstellung eines Tampons gemäß Anspruch 8, dadurch gekennzeichnet, daß man einen Kabelabschnitt der Breite von etwa 2 bis 6 cm verwendet.

10. Verfahren zur Herstellung eines Tampons, gemäß einem der Ansprüche 8 bis 9, dadurch gekennzeichnet, daß man zwischen Schritt i) und ii) einen Rückholfaden etwa in der Mitte der Längsseite des rechteckigen Kabelabschnitts einbringt, so daß dieser Faden wesentlichen senkrecht zur Lage der Einzelfilamente verläuft.

11. Verfahren zur Herstellung eines Tampons gemäß Anspruch 10, dadurch gekennzeichnet, daß die Befestigung des Rückholfadens durch Umschlingen des Kabelabschnitts erfolgt.

12. Verfahren zur Herstellung eines Tampons gemäß einem der Ansprüche 8 bis 11, dadurch gekennzeichnet, daß der aufgerollte Kabelabschnitt nach Schritt ii) mit einem Hüllvlies versehen wird.

| | **EINSCHLÄGIGE DOKUMENTE** | | EP 91104873.4 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl.⁵) |
| A | US - A - 4 351 339 (SNEIDER) * Gesamt * -- | 1 | A 61 F 13/20 |
| A | GB - A - 2 211 096 (SMITH & NEPHEW) * Anspruch 1 * -- | 1 | |
| A | AT - B - 257 035 (DR. HAHN) * Gesamt * ---- | 1,8 | |
| | | | **RECHERCHIERTE SACHGEBIETE (Int. Cl.⁵)** |
| | | | A 61 F 13/00 |
| | Der vorliegende Recherchenbericht wurde für alle Patentansprüche erstellt. | | |

| Recherchenort | Abschlußdatum der Recherche | Prüfer |
|---|---|---|
| WIEN | 11-06-1991 | KNAUER |